# EUROPEAN PATENT SPECIFICATION

(11) **EP 3 076 926 B1**
(45) Date of publication and mention of the grant of the patent: **14.02.2018**
(21) Application number: 13824693.9
(22) Date of filing: 05.12.2013
(51) Int. Cl.: A61K 8/34, A61Q 11/00, A61K 8/22, A61K 8/24, A61K 8/46, A61K 8/49, A61K 8/81, A61K 8/86, A61K 8/19

(54) **ORAL CARE WHITENING COMPOSITIONS CONTAINING FATTY AMPHIPHILES**
AUFHELLUNGSMITTEL MIT FETTSÄUREAMPHIPHILEN FÜR DIE MUNDPFLEGE
COMPOSITIONS DE BLANCHIMENT POUR L'HYGIÈNE BUCCALE CONTENANT DES AMPHIPHILES GRAS

(43) Date of publication of application: 12.10.2016
(73) Proprietor: Colgate-Palmolive Company, New York, NY 10022 (US)
(72) Inventor: PAN, Guisheng, Philadelphia, PA 19114 (US); MANDADI, Prakasarao, Flemington, NJ 08822 (US); FEI, Lin, Kendall Park, NJ 08824 (US); CHOPRA, Suman K., Monroe, New Jersey 08831 (US)
(74) Representative: Wibbelmann, Jobst
(86) International application number: PCT/US2013/073234
(87) International publication number: WO 2015/084357

(56) References cited:
- WO-A1-2014/092736
- US-A1- 2006 062 744
- US-A1- 2007 071 695
- US-A1- 2008 081 023
- US-A1- 2013 108 559

## Description

### BACKGROUND

Dentifrice formulations comprising peroxide are known and useful for cleaning and whitening teeth. The peroxide can bleach the teeth, remove stains, and kill cariogenic bacteria. However, peroxide compounds are highly reactive, and consequently difficult to formulate. Moreover, hydrogen peroxide can spontaneously decompose to form oxygen gas (O₂) and water, so that on storage, the dentifrice containers may bloat, burst or leak, and the remaining formulation will not have enough peroxide remaining to clean and whiten teeth effectively. Some dentifrices initially comprise very high levels of peroxide, which decomposes over time, so that the exact amount of peroxide delivered on application is variable and largely depends on how long and under what conditions the dentifrice has been stored.

To solve the H₂O₂ stability problems, improved dentifrices using stabilized H₂O₂ have been used which comprise H₂O₂ complexed with polyvinylpyrrolidone. (PVP). By exposure to aqueous environments, as in the oral cavity, the PVP-H₂O₂ dissociates into individual species (PVP polymer and H₂O₂). The PVP-H₂O₂ complex is generally comprised of about 80% by weight polyvinyl pyrrolidone and 20% by weight H₂O₂. PVP-H₂O₂ complexes and/or single phase whitening dentifrice compositions comprising same are described, e.g., in WO/2007/037961, US Pub. No. US 2007-0071695 A1, US Pub. No. US 2012-0058059 A1, and US Pat. No. 5,122,370.

Phase separation is a significant challenge for gel dentifrices containing peroxides. Bloating can also be a problem in gel dentifrices containing peroxides.

There is thus a need for improved peroxide dentifrice formulations that are stable for long-term storage and are suitable for everyday consumer use.

### SUMMARY

The present invention provides dentifrice compositions as defined in the claims that are single phase dentifrices that can be facilely dispensed by the consumer from a single chamber tube. In some embodiments the dentifrice compositions are stable during long term storage and remain effective to clean and whiten teeth. The dentifrice composition comprises: (i) a whitening complex comprising a crosslinked polyvinylpyrrolidone complexed with hydrogen peroxide and (ii) a fatty amphiphile as defined in the claims. The fatty amphiphile is stearyl alcohol, cetyl alcohol or a combination of both

Further embodiments of the invention will be apparent from the detailed description and the examples.

### DETAILED DESCRIPTION

As used throughout, ranges are used as shorthand for describing each and every value that is within the range. Any value within the range can be selected as the terminus of the range. Unless otherwise specified, all percentages and amounts expressed herein and elsewhere in the specification should be understood to refer to percentages by weight. All percentages expressed herein are on a weight by dry matter basis unless specifically stated otherwise

In the event of a conflict in a definition in the present disclosure and that of a cited reference, the present disclosure controls.

As referred to herein, an "oral care composition" is any composition that is suitable for administration or application to the oral cavity of a human or animal subject for enhancing the health, hygiene or appearance of the subject. In some embodiments, an oral care composition is retained in the oral cavity for a time sufficient to affect the intended utility.

The oral care composition is a dentifrice composition. By way of example and not limitation, the term "dentifrice" as used throughout this description, denotes a paste, gel, toothpowder, dental tablet or liquid formulation. In some embodiments, the dentifrice deep striped, surface striped, or multilayered, having a gel surrounding the paste. In some embodiments, the composition is used with a tape, tray, mouthpiece or similar appliance. In a preferred embodiment the oral composition is a single phase toothpaste or gel.

As used herein, the phrase "unacceptable level of phase separation" means phase separation that can be observed by the unaided eye after storage for 24 hours

Also disclosed are oral care compositions comprising (i) a crosslinked polyvinylpyrrolidone complexed with hydrogen peroxide, (ii) a fatty amphiphile, and (iii) a carrier comprising an ethylene oxide, propylene oxide co-polymer having an average molecular weight of greater than 1000 Da.

Further disclosed are oral care compositions comprising (i) a crosslinked polyvinylpyrrolidone complexed with hydrogen peroxide, (ii) a fatty amphiphile, and (iii) a carrier comprising (a) an ethylene oxide, propylene oxide block co-polymer of formula (ethylene oxide)ₓ-(propylene oxide)_{y} wherein x is an integer of 80-150 and y is an integer 30-80, having an average molecular weight of greater than 5000 Da, and (b) an abrasive. In some embodiments, the abrasive is a calcium abrasive. In some embodiment the composition further comprises a surfactant. In some embodiments the composition further comprises a humectant. In some embodiments the composition further comprises a solvent. In other embodiments, the invention provides an abrasive-free gel.

In one embodiment, the dentifrice composition is Composition 1, which is a toothpaste comprising (i) a whitening complex comprising crosslinked polyvinylpyrrolidone complexed with hydrogen peroxide, (ii) a fatty amphiphile as defined in the claims, e.g.,
1.1. Composition 1 wherein the whitening complex contains about 10-30%, e.g., 15-25%, for example about 17-22% of hydrogen peroxide by weight, and about 5-15%, for example about 7-12% total nitrogen by weight; for example, having substantially the same specifications as Polyplasdone® XL-10, e.g., Polyplasdone® XL-10F, e.g., available from International Specialty Products (Wayne, NJ);
1.2. Any of the foregoing compositions further comprising a calcium abrasive such as calcium carbonate or calcium pyrophosphate;
1.3. Any of the foregoing compositions wherein the total amount of hydrogen peroxide by weight of the composition is 0.5-5%, e.g., 0.75 -3%, e.g. about 1%;
1.4. Any of the foregoing compositions which contains less than 10 water e.g., less than 2% water, or less than 1% water, e.g., is substantially anhydrous;
1.5. Any of the foregoing compositions further comprising polymer thickeners selected from (i) polyethylene glycol, (ii) polyethylene glycol - polypropylene glycol co-polymers having a molecular weight of at least 1000, and (iii) combinations thereof;
1.6. The preceding composition comprising an ethylene oxide, propylene oxide co-polymer having an average molecular weight of greater than 1000, e.g. 5000 - 13000 Da, e.g. about 9800;
1.7. The preceding composition additionally comprising polyethylene glycol of average molecular weight 400 to 800, e.g., about 600 Da;
1.8. Any of the foregoing compositions additionally comprising humectants, e.g. selected from glycerin, propylene glycol or a combination thereof;
1.9. Any of the foregoing compositions additionally comprising a tartar control agent, e.g., selected from tetrasodium pyrophosphate (TSPP) and sodium tripolyphosphate (STPP);
1.10. Any of the foregoing compositions additionally comprising a surfactant, e.g., sodium lauryl sulfate (SLS);
1.11. Any of the foregoing compositions additionally comprising an antibacterial agent, e.g., triclosan;
1.12. Any of the foregoing compositions additionally comprising an antioxidant, e.g., butylated hydoxytoluene (BHT);
1.13. Any of the foregoing compositions additionally comprising a fluoride ion source such as sodium fluoride or sodium monofluorophosphate ;
1.14. Any of the foregoing compositions comprising any or all of the following ingredient classes and/or particular ingredients by weight:

| | |
|---|---|
| Solvents | |
| Glycerin | 0-7%, e.g., 3-7% or about 5% |
| Propylene glycol | 20-70%, e.g., about 40-65%, or about 50-65%, or about 50-60% |
| Ethylene oxide, propylene oxide co-polymer, avg. MW >1kDa | 5-15%, e.g., about 7-10% orabout 7.5% |
| Polyethylene glycol 600 | 0-15%, e.g., about 10% |
| Thickeners, e.g., | |
| Fumed silica | 0-5%, e.g., about 1.5% |
| Polyvinylpyrrolidone | 0-10% |
| Whitener, 3-15%, e.g., | |
| Crosslinked polyvinylpyrrolidone complexed with 15-25% hydrogen peroxide | 3-22%, e.g., about 5-12% or about 9-12% |
| Abrasive, 5-45%, e.g. | |
| Calcium pyrophosphate | 5-45% or 10-35%, e.g., about 15% |
| Fluoride, 0 - 1%, e.g. | |
| Sodium monofluorophosphate | 0.5 - 1%, e.g., about 0.76% |
| Surfactant, e.g., SLS | 0-5 e.g., 0-3%, e.g.,.1-3%, e.g., about 2% |
| Antioxidant, 0.01-5%, e.g. | |
| BHT | 0.01-0.05%, e.g., 0.03% |
| Flavorings | 0.1 - 5% |
| Water | <3% |
| Tartar control agent, e.g. TSPP | 0.1-5%, or 0.3-5%, e.g., about 0.3-0.5% |

1.15. The composition resulting from the combination of the preceding ingredients.

Disclosed are oral care compositions comprising: a crosslinked polyvinylpyrrolidone complexed with hydrogen peroxide, a fatty amphiphile, a solvent, a polymer thickener an abrasive and a humectant.

Some embodiments provide oral care compositions comprising: from about 0.5 to about 22%, by weight, crosslinked polyvinylpyrrolidone complexed with hydrogen peroxide. Other embodiments provide oral care compositions comprising from about 1 to about 15%, by weight, crosslinked polyvinylpyrrolidone complexed with hydrogen peroxide. Still other embodiments provide oral care compositions comprising from about 3 to about 15%, by weight, crosslinked polyvinylpyrrolidone complexed with hydrogen peroxide. Yet other embodiments provide oral care compositions comprising from about 4 to about 12%, by weight, crosslinked polyvinylpyrrolidone complexed with hydrogen peroxide. While other embodiments provide oral care compositions comprising from about 5 to about 11%, by weight, crosslinked polyvinylpyrrolidone complexed with hydrogen peroxide. In some embodiments, the oral care compositions comprise about 5%, 6%, 7%, 8%, 8%, 9%, 10%, 11% or 12%, by weight, crosslinked polyvinylpyrrolidone complexed with hydrogen peroxide. In some embodiments, the oral care compositions comprise 5.5% by weight, crosslinked polyvinylpyrrolidone complexed with hydrogen peroxide.

The compositions of the present invention comprise a fatty amphiphile as defined in the claims. As used herein, "fatty amphiphile" refers to a compound having a hydrophobic tail group of R₁ as defined below and a hydrophilic head group which does not make the compound water soluble (immiscible), wherein the compound also has a net neutral charge at the pH of the oral composition. The term "water soluble", as used herein, means that the material is soluble in water in the present composition. In general, the material should be soluble at 25° C. at a concentration of 0.1% by weight of the water solvent, preferably at 1%, more preferably at 5%, more preferably at 15%.

The fatty amphiphile of the present invention is defined in the claims. A fatty amphipile may be characterized as a compound having a Hydrophilic-Lipophilic Balance ("HLB") of 6 or less. The HLB, as used herein, is the standard HLB according to Griffin, J. Soc. Cosm. Chem., vol. 5, 249 (1954). If using a mixture of fatty amphiphiles, it is desired that the mixture have a HLB of from about 1 to about 6 and preferably from about 1 to about 3. Therefore, fatty amphiphile having an HLB above 6 can be used if it is mixed with another fatty amphiphile having a lower HLB.

The oral compositions of the present invention comprise fatty amphiphile in an amount from 0.1% to 20%, and more preferably from 0.5 % to 10%, by weight of the final oral composition. In some examples, the amount of fatty amphiphile in the final oral composition is from about 2% to about 8% and more preferably from about 4% to about 6%.

Fatty amphiphiles preferably have a melting point of at least about 40° C. The melting point, as used herein, may be measured by a standard melting point method as described in U.S. Pharmacopeia, USP-NF General Chapter 741 "Melting range or temperature". The melting point of a mixture of two or more materials is determined by mixing the two or more materials at a temperature above the respective melt points and then allowing the mixture to cool. If the resulting composite is a homogeneous solid below about 45° C., then the mixture has a suitable melting point for use in the present invention.

Fatty amphiphiles can have a hydrophobic tail group of R₁. As used herein, R₁ is an alkyl, alkenyl (containing up to 3 double bonds), alkyl aromatic, or branched alkyl group of C₁₂-C₇₀ length. Non-limiting examples of alkyl, alkenyl, or branched alkyl groups suitable for the fatty amphiphiles include lauryl, tridecyl, myristyl, pentadecyl, cetyl, heptadecyl, stearyl, arachidyl, behenyl, undecylenyl, palmitoleyl, oleyl, palmoleyl, linoleyl, linolenyl, arahchidonyl, elaidyl, elaeostearyl, erucyl, isolauryl, isotridecyl, isomyristal, isopentadecyl, petroselinyl, isocetyl, isoheptadecyl, isostearyl, isoarachidyl, isobehnyl, gadoleyl, brassidyl, and technical-grade mixture thereof. As used herein, R₁ also may be a branched alkyl group prepared by alkaline condensation of alcohols to give higher molecular weight, branched isoalcohols. These branched isoalcohols are referred to in the art as Guerbet alcohols. R₁ may be alkyl, alkenyl or branched carbon chains of vegetable origin, such as wheat germ, sunflower, grape seed, sesame, maize, apricot, castor, avocado, olive, soybean, sweet almond, palm, rapeseed, cotton seed, hazelnut, macadamia, karite, jojoba, alfalfa, poppy, pumpkinseed, sesame, cucumber, blackcurrant, evening primrose, millet, barley, quinoa, rye, safflower, candlenut, passion flower or musk rose oil, and karite butter.

Fatty amphiphiles can also have a hydrophilic head group which does not make the compound water soluble, such as in compounds having an HLB of 6 or less. Non-limiting examples of classes of compounds having such a hydrophilic head group include fatty alcohols, alkoxylated fatty alcohols, fatty phenols, alkoxylated fatty phenols, fatty amides, alkyoxylated fatty amides, fatty amines, fatty alkylamidoalkylamines, fatty alkyoxyalted amines, fatty carbamates, fatty amine oxides, fatty acids, alkoxylated fatty acids, fatty diesters, fatty sorbitan esters, fatty sugar esters, methyl glucoside esters, fatty glycol esters, mono, di & tri glycerides, polyglycerine fatty esters, alkyl glyceryl ethers, propylene glycol fatty acid esters, cholesterol, ceramides, fatty silicone waxes, fatty glucose amides, and phospholipids.

Fatty amphiphiles can be fatty alcohol compounds or alkoxylated fatty alcohol ether compounds according to the following formula:

R₁-(OR₂)ₖ-OH

wherein R₁ is as described above; R₂ is a C₁-C₅ carbon chain which may be branched or hydroxy substituted; and k is a number ranging from about 0 to about 5. The fatty alcohols useful herein are those having from about 12 to about 60 carbon atoms, preferably from about 16 to about 60 carbon atoms. These fatty alcohols may be straight or branched chain alcohols and may be saturated or unsaturated. Non-limiting examples of suitable fatty alcohols include cetyl alcohol, stearyl alcohol, arachidyl alcohol, behenyl alcohol, eicosyl alcohol, C20-40 alcohols, C30-50 alcohols, C40-60 alcohols, and mixtures thereof. Suitable alkoxylated fatty alcohol ethers include addition products of 1 to 5 mol of ethylene oxide with a linear fatty alcohol having about 12 to about 60 carbon atoms, which are all adducts obtainable by the known industrial oxyethylation processes. Also suitable are the polyethylene oxide condensates of alkyl phenols, for example, the condensation products of alkyl phenols having an alkyl group containing from about 12 to about 60 carbon atoms in either a straight chain or branched chain configuration, with ethylene oxide, wherein the ethylene oxide is present in amounts equal to from about 1 to about 5 moles of ethylene oxide per mole of alkyl phenol. Further suitable alkoxylated fatty alcohol ethers include those derived from the condensation of ethylene oxide with the product resulting from the reaction of propylene oxide and ethylene diamine products. Non-limiting examples of suitable alkoxylated fatty alcohol ethers include steareth-2, beheneth-2, beheneth-5, beheneth-10, C20-40 Pareth-3, C20-40 Pareth-10, C30-50 Pareth-3, and C30-50-Pareth-10.ln one embodiment, a combination of fatty alcohols such as cetyl and stearyl alcohol is preferred. The ratio of cetyl to stearyl alcohol can be from about 4:1 to about 1:4, preferably from about 2:1 to about 1:2, and in some embodiments 1:1.Fatty amphiphiles also may be di-fatty ethers, fatty amides including fatty alkanolamides and fatty alkoxylated amides, fatty carbamates, fatty alkylamido alkylamines, fatty amines including fatty alkanolamines and fatty alkoxylated amines, fatty amine oxides, fatty acids or alkoxylated fatty acids, fatty esters, fatty phosphorus compounds fatty sorbitan derivatives, sucrose polyesters, alkyl sulfoxides, and combinations thereof.

The compositions of the present invention may comprise a surface active agent (surfactant). Suitable surfactants include anionic, zwitterionic, amphoteric, cationic, and nonionic surfactants. The surfactants may be a combination of more than one type of surfactants, such as an anionic and nonionic surfactant. The surfactant is typically water soluble or miscible in the solvent or oral carrier. In one embodiment, anionic surfactants such as sodium lauryl sulfate, are preferred. Suitable surfactants include without limitation water-soluble salts of C₈₋₂₀ alkyl sulfates, sulfonated monoglycerides of C₈₋₂₀ fatty acids, sarcosinates, taurates, sodium lauryl sulfate (SLS), sodium cocoyl monoglyceride sulfonate, sodium lauryl sarcosinate, sodium lauryl isoethionate, sodium laureth carboxylate and sodium dodecyl benzenesulfonate, and cocoamidopropyl betaine. The surfactant is typically present in an amount from about 0.01% to about 15%, in another embodiment from about 0.1% to about 10%, and in another embodiment from about 0.3% to about 5%, by weight of the oral composition. In some embodiments, a diluted solution of surfactant in water is utilized. In one embodiment, the amount of surfactant is chosen based on the level of foaming desired in the oral composition and on the irritation caused by the surfactant.

In some embodiments the compositions of the invention also comprise solvents, such as water or other suitable solvents, such as humectants. Suitable solvents for the present invention include water, edible polyhydric alcohols such as glycerin, diglycerin, triglycerin, sorbitol, xylitol, butylene glycol, erythritol, polyethylene glycol, propylene glycol, and combinations thereof. Sorbitol, glycerin, and combinations thereof are preferred solvents

The oral compositions may comprise at least about 0.05% of a solvent, by weight of the oral composition. The solvent may be present in the oral composition in amount of from about 0.1% to about 99%, from about 0.5% to about 95%, and from about 1% to about 90%.

If the viscosity of an oral composition is too low (thin or less viscous), the composition will not suitable for use as a toothpaste; conversely if the viscosity is too high (thick or more viscous), the composition will also not be suitable for use as a toothpaste. The compositions of the invention typically have a pH of about 5 to about 9, more particularly about 6 to about 8, and more particularly about 7.

The compositions of the invention also typically comprise an orally acceptable carrier or vehicle. The carrier may comprise abrasives, thickening agents, humectants, other polymers, colorants, viscosity modifiers, foam modulators, emulsifiers, pH modifying agents, diluents, mouth feel agents, sweetening agents, flavor agents, preservatives, suitable cosmetic and/or therapeutic actives, and combinations thereof. It is understood that while general attributes of each of the above categories of materials may differ, there may be some common attributes and any given material may serve multiple purposes within two or more of such categories of materials. All of the ingredients in the compositions may have functions in addition to their primary function, and may contribute to the overall properties of the composition, including its stability, efficacy, consistency, mouthfeel, taste, odor and so forth. Preferably, the carrier is selected for compatibility with other ingredients of the composition.

Actives include any material that is generally considered safe for use in the oral cavity which is operable for the prevention or treatment of a condition or disorder of hard or soft tissue of the oral cavity, the prevention or treatment of a physiological disorder or condition, or to provide a cosmetic benefit.changes to the overall appearance and/or health of the oral cavity. Examples of actives include, but are not limited to, anti-calculus agents, fluoride ion sources, stannous ion sources, other whitening agents, anti-microbial agents, anti-malodor agents, anti-sensitivity agents, anti-erosion agents, anti-caries agents, anti-plaque agents, anti-inflammatory agents, saliva-stimulating agents, nutrients, antioxidants, anti-viral agents, analgesic and anesthetic agents, H-2 antagonists, and mixtures thereof. When present, the level of cosmetic and/or therapeutic active in the oral care composition is, in one embodiment is from about 0.001% to about 90%, in another embodiment from about 0.01% to about 50%, and in another embodiment from about 0.1% to about 30%, by weight of the oral care composition.

It has been found that additional linear and/or crosslinked polyvinylpyrrolidone, i.e., in addition to the PVP that is part of the PVP-H₂O₂ complex, is not necessary to provide stable or single phase compositions. However, in some embodiments additional linear and/or crosslinked polyvinylpyrrolidone can be added from about 1 to about 15%, by weight.

Some embodiments of the present invention as defined in the claims provide gel-based peroxide oral compositions further comprising a calcium abrasive. In some embodiments, the compositions comprise from about 9 to about 25%, by weight, propylene glycol. In some embodiments, the compositions comprise from about 14 to about 32%, by weight, glycerin. In other embodiments, the compositions comprise less than 20%, by weight, of a calcium abrasive. Some embodiments provide compositions comprising from about 9 to about 25%, by weight, propylene glycol; from about 14 to about 32%, by weight, glycerin; and less than 20%, by weight, of a calcium abrasive.

Still other embodiments provide oral care compositions as defined in the claims comprising from about 20 to about 60%, by weight, humectant.

Yet further embodiments provide oral care compositions as defined in the claims comprising from about 5 to about 15%, by weight, abrasive.

The compositions of the invention are "low water" content, meaning that a total concentration of water, including any free water and all water contained in any ingredients, is less than about 20% total water, or less than about 10% total water, in another embodiment, less than about 5%, in another embodiment less than 3%, in another embodiment less than 2% water, in another embodiment less that 1% water, i.e., is anhydrous.

Where abrasives are present, the average particle size is generally about 0.1 to about 30 microns, for example about 1 to about 20 or about 5 to about 15 microns.

In various embodiments of the present invention, the oral composition comprises an anticalculus (tartar control) agent. Generally, tartar control agents are categorized as being incompatible with some whitening agents, but embodiments of the present invention incorporate tartar control agents and whitening agents in a single phase whitening composition. Suitable anticalculus agents include without limitation phosphates and polyphosphates (for example pyrophosphates), polyaminopropanesulfonic acid (AMPS), hexametaphosphate salts, zinc citrate trihydrate, polypeptides, polyolefin sulfonates, polyolefin phosphates, diphosphonates. The anticalculus agent is present at about 0.1% to about 30%. The oral composition may include a mixture of different anticalculus agents. In one preferred embodiment, tetrasodium pyrophosphate (TSPP) and sodium tripolyphosphate (STPP) are used. In one embodiment the anticalculus agent comprises TSPP at about 1-2% and STPP at about 7% to about 10%.

The oral care composition can optionally include at least one orally acceptable source of fluoride ions. Any known or to be developed in the art may be used, e.g., soluble fluoride salts. A wide variety of fluoride ion-yielding materials can be employed as sources of soluble fluoride in the present compositions. Examples of suitable fluoride ion-yielding materials are found in U.S. Pat, No. 3,535,421, to Briner et al.; U,S, Pat. No. 4,885,155, to Parian, Jr. et al. and U.S. Pat. No. 3,678,154, to Widder et al. Representative fluoride ion sources include, but are not limited to, stannous fluoride, sodium fluoride, potassium fluoride, sodium monofluorophosphatc, sodium fluorosilicate, ammonium fluorosilicate, amine fluoride, ammonium fluoride, and combinations thereof In certain embodiments the fluoride ion source includes stannous fluoride, sodium fluoride, sodium monofluorophosphatc as well as mixtures thereof. In certain embodiments, the oral care composition of the invention may also contain a source of fluoride ions or fluorine-providing ingredient in amounts sufficient to supply about 25 ppm to about 25,000 ppm of fluoride ions, generally at least about 500 ppm, e.g., about 500 to about 2000 ppm, e.g., about 1000 to about 1600 ppm, e.g., about 1450 ppm. The appropriate level of fluoride will depend on the particular application. A toothpaste for general consumer use would typically have about 1000 to about 1500 ppm, with pediatric toothpaste having somewhat less, in other embodiments the level of fluoride is about 100 to about 20,000 ppm, about 200 to about 5,000 ppm, or about 500 to about 2,500 ppm, fluoride ions. A dentifrice or coating for professional application could have as much as about 5,000 or even about 25,000 ppm fluoride. Fluoride ion sources may be added to the compositions of the invention at a level of about 0.01 wt. % to about 10 wt. % in one embodiment or about 0.03 wt. % to about 5 wt. %, and in another embodiment about 0.1 wt. % to about 1 wt. % by weight of the composition in another embodiment. Weights of fluoride salts to provide the appropriate level of fluoride ion will obviously vary based on the weight of the counter ion in the salt.

The compositions of the invention may also comprise various dentifrice ingredients to adjust the rheology and feel of the composition such as humectants, surface active agents, thickening or gelling agents, etc.

The compositions of the present invention optionally comprise a thickener. Any orally acceptable thickening agent can be used, including without limitation carbomers, also known as carboxyvinyl polymers, carrageenans, also known as Irish moss and more particularly - carrageenan (iota-carragecnan), high molecular weight polyethylene glycols (such as CARBOWAX®, available from The Dow Chemical Company), cellulosic polymers such as hydroxyethylcellulose, carboxymcthylcellulosc (CMC) and salts thereof, e.g., CMC sodium, natural gums such as karaya, xanthan, gum arable and tragacanth, colloidal magnesium aluminum silicate, and colloidal and/or fumed silica and mixtures of the same. One or more thickening agents are optionally present in a total amount of about 0.1 % to about 90%, for example about 1% to about 50% or about 5% to about 35%.

In various preferred embodiments, the carrier may comprise polymers and/or copolymers of polyethylene glycol, of ethylene oxide/propylene oxide, and of silicone. If such copolymers/polymers are used, they may be selected from commercially available materials. Block copolymers of ethylene oxide/propylene oxide are useful, but higher molecular weight, e.g., > 1000Da are preferred, e.g. including PLURACARE® L1220 (available from BASF, Wyandotte, Mich., United States of America). Low or medium molecular weight polyethylene glycol, e.g., PEG 400, PEG 600, PEG 800, PEG 1000 and mixtures thereof arc also useful.

The compositions may include a stannous ion or a stannous ion source. Suitable stannous ion sources include without limitation stannous fluoride, other stannous halides such as stannous chloride dihydrate, stannous pyrophosphate, organic stannous carboxylate salts such as stannous formate, acetate, gluconate, lactate, tartrate, oxalate, malonatc and citrate, stannous ethylene giyoxi.de and the like. One or more stannous ion sources are optionally and illustratively present in a total amount of about 0.01% to about 10%, for example about 0.1% to about 7% or about 1% to about 5%.

The compositions of the present invention optionally comprise an antimicrobial (e.g., antibacterial) agent. A further illustrative list of useful antibacterial agents is provided in such as those listed in U.S. Pat. No. 5,776,435 to Gaffar et al. One or more antimicrobial agents are optionally present in an antimicrobial effective total amount, typically about 0.05% to about 10%, for example about 0,1% to about 3%.

The compositions of the present invention optionally comprise an antioxidant. Any orally acceptable antioxidant can be used, including butylatcd hydroxyanisolc (BHA), butylated hydroxytolucnc (BHT), vitamin A, carotenoids, vitamin E, flavonoids, polyphenols, ascorbic acid, herbal antioxidants, chlorophyll, melatonin, and mixtures thereof.

Methods arc provided to whiten an oral surface in a human or animal subject comprising storing in stable form a composition of the invention, e.g.. Composition 1, *et seq,,* as described above, and contacting said composition with the oral surface. As used herein "animal subject" includes higher order non-human mammals such as canines, felines, and horses. The oral care composition is contacted with an oral surface of the mammalian subject to thereby whiten teeth in a highly efficacious manner, without any negative interaction between the whitening agent, the peroxide incompatible abrasive, and other ingredients.

In various embodiments, it is preferred that the oral care composition is applied and contacted with the oral surface. The dentifrice, prepared in accordance with the present invention is preferably applied regularly to an oral surface, preferably on a daily basis, at least one time daily for multiple days, but alternately every second or third day. Preferably the oral composition is applied to the oral surfaces from 1 to 3 times daily, for at least 2 weeks up to 8 weeks, from four months to three years, or more up to lifetime.

In some embodiments, the compositions of the present invention do not exhibit an unacceptable level of phase separation after storage at room temperature for 24 hours.. In other embodiments, the compositions of the present invention do not exhibit an unacceptable level of phase separation after storage at room temperature for 48 hours, 72 hours, 200 hours, 1 week, 1 month, 3 months, or six months.

In some embodiments, the diameter of the top of the tube in which a composition of the present invention is packaged, expands less than 0.01% of the top diameter of the tube, after 1 week of aging at 60°C. In some embodiments, the diameter of the top of the tube in which a composition of the present invention is packaged, expands less than 0.05% of the top diameter of the tube, after 1 week of aging at 60°C. In some embodiments, the diameter of the top of the tube in which a composition of the present invention is packaged, expands less than 0.1% of the top diameter of the tube, after 1 week of aging at 60°C. In some embodiments, the diameter of the top of the tube in which a composition of the present invention is packaged, expands less than 0.5% of the top diameter of the tube, after 1 week of aging at 60°C. In some embodiments, the diameter of the top of the tube in which a composition of the present invention is packaged, expands less than 1%) of the top diameter of the tube, after 1 week of aging at 60°C. In some embodiments, the diameter of the top of the tube in which a composition of the present invention is packaged, expands less than 3% of the top diameter of the tube, after 1 week of aging at 60°C. In some embodiments, the diameter of the top of the tube in which a composition of the present invention is packaged, expands less than 5% of the top diameter of the tube, after 1 week of aging at 60°C. In some embodiments, the diameter of the top of the tube in which a composition of the present invention is packaged, expands less than 10% of the top diameter of the tube, after 1 week of aging at 60°C. In some embodiments, the diameter of the top of the tube in which a composition of the present invention is packaged, expands less than 15% of the top diameter of the tube, after 1 week of aging at 60°C. In some embodiments, the diameter of the top of the tube in which a composition of the present invention is packaged, expands less than 20% of the top diameter of the tube, after 1 week of aging at 60°C.

In some embodiments, the diameter of the top of the tube in which a composition of the present invention is packaged, expands less than 25% of the top diameter of the tube, after 1 week of aging at 60°C. In other embodiments, the diameter of the top of the tube in which a composition of the present invention is packaged, expands less than 30% of the top diameter of the tube, after 1 week of aging at 60°C. In some embodiments, the diameter of the top of the tube in which a composition of the present invention is packaged, expands less than 35% of the top diameter of the tube, after 1 week of aging at 60°C. In some embodiments, the diameter of the top of the tube in which a composition of the present invention is packaged, expands less than 40% of the top diameter of the tube, after 1 week of aging at 60°C. In some embodiments, the diameter of the top of the tube in which a composition of the present invention is packaged, expands less than 45% of the top diameter of the tube, after 1 week of aging at 60°C. In some embodiments, the diameter of the top of the tube in which a composition of the present invention is packaged, expands less than 50% of the top diameter of the tube, after 1 week of aging at 60°C.

The invention is illustrated in the following non-limiting examples.

### EXAMPLES

### Example 1

### Preparation of gel dentifrices

1. Dissolve BHT in flavor
2. Add Pluorocare L1220 and propylene glycol liquids in a stainless steel container
3. Weigh, Na saccharin, sucralose, Na monofluorophosphate, TSPP, and SLS, and put into liquids from step 2
4. Heat the resulting mixture to above 75°C
5. Melt stearyl alcohol or cetyl alcohol (if used) into another beaker, above 75°C
6. Weigh the hot fatty alcohol from step 5 and quickly pour into the mixture from step 4
7. Keep stirring the resulting mixture for 15 minutes, stop heat, transfer into a ross pot
8. Add Ca₂P₂O₇ to the ross pot
9. Stir the mixture at full speed and apply full vacuum in ross pot until the mixture reaches room temperature (about 30 minutes).
10. Add the crosslinked PVP/H₂O₂ and flavor/BHT from step 1, stir and apply full vacuum for 10 minutes
11. Transfer the final product in a sealed glass container.

### Example 2

A gel dentifrice having a peroxide composition of about 2% is prepared having the ingredients in Table 1.

**Table 1**

| **Ingredient** | **% (w/w)** |
|---|---|
| Propylene glycol | 53.51 |
| PEG₁₁₈/PPG₆₆ co-polymer (Plurocare L1220) | 7.5 |
| Crosslinked polyvinylpyrrolidone-hydrogen peroxide | 11 |
| Sodium saccharine | 0.6 |
| Sucralose | 0.05 |
| Sodium monofluorophosphate | 0.76 |
| TSPP | 0.3 |
| BHT | 0.03 |
| Flavor | 2.25 |
| Sodium lauryl sulfate | 2 |
| Stearyl alcohol | 7 |
| Cetyl alcohol | 0 |
| Ca₂P₂O₇ | 15 |
| Total | 100 |

### Example 3

A gel dentifrice having a peroxide composition of about 2% is prepared having the ingredients in Table 2.

**Table 2**

| **Ingredient** | **% (w/w)** |
|---|---|
| Propylene glycol | 53.51 |
| PEG₁₁₈/PPG₆₆ co-polymer (Plurocare L1220) | 7.5 |
| Crosslinked polyvinylpyrrolidone/hydrogen peroxide | 11 |
| Sodium saccharine | 0.6 |
| Sucralose | 0.05 |
| Sodium monofluorophosphate | 0.76 |
| TSPP | 0.3 |
| BHT | 0.03 |
| Flavor | 2.25 |
| Sodium lauryl sulfate | 2 |
| Stearyl alcohol | 0 |
| Cetyl alcohol | 7 |
| Ca₂P₂O₇ | 15 |
| Total | 100 |

### Example 4

A toothpaste having a peroxide content of about 2% is prepared with the ingredients in Table 3.

**Table 3**

| **Ingredient** | **% (w/w)** |
|---|---|
| Polyethylene glycol | 60.51 |
| PEG₁₁₈/PPG₆₆ co-polymer (Plurocare L1220) | 7.5 |
| Crosslinked polyvinylpyrrolidone/hydrogen peroxide | 11 |
| Sodium saccharine | 0.6 |
| Sucralose | 0.05 |
| Sodium monofluorophosphate | 0.76 |
| TSPP | 0.3 |
| BHT | 0.03 |
| Flavor | 2.25 |
| Sodium lauryl sulfate | 2 |
| Stearyl alcohol | 0 |
| Cetyl alcohol | 0 |
| Ca₂P₂O₂ | 15 |
| Total | 100 |

### Example 5

*Physical separation:* The toothpastes of Example 2-4 are tested for stability to remain in a single phase. Samples are stored at room temperature for 24 and examined for visible phase separation. Examples 2 and 3, containing stearyl alcohol and cetyl alcohol respectively, are stable in that no phase separation is observed. Example 4 is not stable in that a separation into two distinct phases is observed.

The data described in the Examples evidences the unexpected improvement in chemical and physical stability demonstrated by compositions of the present invention.

## Claims

1. A dentifrice composition comprising (i) a whitening complex comprising crosslinked polyvinylpyrrolidone complexed with hydrogen peroxide, (ii) a fatty amphiphile selected from stearyl alcohol, cetyl alcohol and a combination thereof, wherein the fatty amphiphile is present in an amount of 0.1 to 20 % by weight of the composition.

2. The composition of claim 1 comprising an ethylene oxide, propylene oxide co-polymer of average molecular weight greater than 1000 Da, being substantially free of an ethylene oxide, propylene oxide block co-polymer of average molecular weight less than 1000 Da.

3. The composition of claim 2 wherein the ethylene oxide, propylene oxide co-polymer has an average molecular weight of between 5000 and 13000 Da.

4. The composition of any of the preceding claims wherein the whitening complex contains 10-30% hydrogen peroxide, by weight, and 5-15% total nitrogen by weight.

5. The composition of any of the preceding claims wherein the total amount of hydrogen peroxide by weight of the composition is 0.5-5%.

6. The composition of any of the preceding claims additionally comprising polyethylene glycol of average molecular weight 400 to 800 Da.

7. The composition of any of the preceding claims which contains less than 3% water.

8. The composition of any of claims 1-7 which is a gel non-abrasive dentifrice.

9. The composition of any of claims 1-7 which is a toothpaste comprising a calcium abrasive selected from a calcium phosphate salt and calcium carbonate.

10. The composition of claim 9 wherein the calcium abrasive comprises calcium pyrophosphate.

11. The composition of any of the preceding claims additionally comprising a tartar control agent such as tetrasodium pyrophosphate.

12. The composition of claim 11 comprising the following ingredients by weight:
a. Glycerin 0-7%
b. Propylene glycol 50-65%
c. Ethylene oxide, propylene oxide co-polymer; avg. MW >1kDa 5-15%
d. Sodium lauryl sulfate 1-3%
e. Crosslinked polyvinylpyrrolidone complexed with 15-25% H₂O₂ 5-12%
f. Calcium pyrophosphate 5-35%
g. tetrasodium pyrophosphate 0.3-5-%%

13. The composition of any of the preceding claims comprising 0.5 - 1% by weight of a fluoride ion source, for example sodium monofluorophosphate.

14. The composition of any of the preceding claims comprising a flavorant, a sweetener, an antioxidant, an antimicrobial or a combination of two or more thereof.

## Patentansprüche

1. Zahnreinigungszusammensetzung, umfassend (i) einen Aufhellungskomplex, der vernetztes Polyvinylpyrrolidon komplexiert mit Wasserstoffperoxid umfasst, (ii) ein Fett-Amphiphiles ausgewählt aus Stearylalkohol, Cetylalkohol und einer Kombination davon, wobei das Fett-Amphiphile in einer Menge von 0,1 bis 20 % bezogen auf das Gewicht der Zusammensetzung vorliegt.

2. Zusammensetzung nach Anspruch 1, die ein Ethylenoxid-Propylenoxid-Copolymer mit einem durchschnittlichen Molekulargewicht von größer als 1000 Da umfasst, die im Wesentlichen frei von einem Ethylenoxid-Propylenoxid-Copolymer mit einem durchschnittlichen Molekulargewicht von weniger als 1000 Da ist.

3. Zusammensetzung nach Anspruch 2, wobei das Ethylenoxid-Propylenoxid-Copolymer ein durchschnittliches Molekulargewicht von zwischen 5000 und 13000 Da aufweist.

4. Zusammensetzung nach einem beliebigen der voranstehenden Ansprüche, wobei der Aufhellungskomplex 10-30% Wasserstoffperoxid bezogen auf das Gewicht und 5-15% Gesamtstickstoff bezogen auf das Gewicht enthält.

5. Zusammensetzung nach einem beliebigen der voranstehenden Ansprüche, wobei die Gesamtmenge an Wasserstoffperoxid bezogen auf das Gewicht der Zusammensetzung 0,5-5% beträgt.

6. Zusammensetzung nach einem beliebigen der voranstehenden Ansprüche, die zusätzlich Polytheylenglycol mit einem durchschnittlichen Molekulargewicht von 400 bis 800 Da umfasst.

7. Zusammensetzung nach einem beliebigen der voranstehenden Ansprüche, die weniger als 3% Wasser enthält.

8. Zusammensetzung nach einem beliebigen der Ansprüche 1-7, die ein nicht abrasives Gel-Zahnreinigungsmittel ist.

9. Zusammensetzung nach einem beliebigen der Ansprüche 1-7, die eine Zahnpasta ist, die einen Calcium-Abrasivstoff umfasst, der aus einem Calciumphosphatsalz und Calciumcarbonat ausgewählt ist.

10. Zusammensetzung nach Anspruch 9, wobei der Calcium-Abrasivstoff Calciumpyrophosphat umfasst.

11. Zusammensetzung nach einem beliebigen der voranstehenden Ansprüche, die zusätzlich ein Zahnstein-Kontrollmittel wie Tetranatriumpyrophosphat umfasst.

12. Zusammensetzung nach Anspruch 11, die die folgenden Bestandteile bezogen auf das Gewicht umfasst:
a. Glycerin 0-7%
b. Propylenglycol 50-65%
c. Ethylenoxid-Propylenoxid-Copolymer, durchschnittl. MW >1kDa 5-15%
d. Natriumlaurylsulfat 1-3%
e. Vernetztes Polyvinylpyrrolidon komplexiert mit 15-25% H₂O₂ 5-12%
f. Calciumpyrophosphat 5-35%
g. Tetranatriumpyrophosphat 0,3-5-%%

13. Zusammensetzung nach einem beliebigen der voranstehenden Ansprüche, die 0,5-1 Gew.-% einer Fluoridionenquelle umfasst, zum Beispiel Natriummonofluorphosphat.

14. Zusammensetzung nach einem beliebigen der voranstehenden Ansprüche, die einen Aromastoff, ein Süßungsmittel, ein Antioxidationsmittel, ein antimikrobielles Mittel oder eine Kombination von zwei oder mehr davon umfasst.

## Revendications

1. Composition de dentifrice comprenant (i) un complexe de blanchiment comprenant de la polyvinylpyrrolidone réticulée complexée avec du peroxyde d'hydrogène, (ii) un amphiphile gras choisi parmi l'alcool stéarylique, l'alcool cétylique ou une combinaison de ceux-ci, dans laquelle l'amphiphile gras est présent en une quantité de 0,1 à 20 % en poids de la composition.

2. Composition selon la revendication 1, comprenant un copolymère d'oxyde d'éthylène et d'oxyde de propylène d'un poids moléculaire moyen supérieur à 1 000 Da, étant sensiblement dépourvu d'un copolymère séquencé d'oxyde d'éthylène et d'oxyde de propylène d'un poids moléculaire moyen inférieur à 1 000 Da.

3. Composition selon la revendication 2, dans laquelle le copolymère d'oxyde d'éthylène et d'oxyde de propylène a un poids moléculaire moyen compris entre 5 000 et 13 000 Da.

4. Composition selon l'une quelconque des revendications précédentes, dans laquelle le complexe de blanchiment contient 10 à 30 % de peroxyde d'hydrogène, en poids, et 5 à 15 % d'azote total en poids.

5. Composition selon l'une quelconque des revendications précédentes, dans laquelle la quantité totale de peroxyde d'hydrogène en poids de la composition est de 0,5 à 5 %.

6. Composition selon l'une quelconque des revendications précédentes, comprenant en outre un polyéthylène glycol d'un poids moléculaire moyen de 400 à 800 Da.

7. Composition selon l'une quelconque des revendications précédentes, qui contient moins de 3 % d'eau.

8. Composition selon l'une quelconque des revendications 1 à 7 qui est un dentifrice non abrasif en gel.

9. Composition selon l'une quelconque des revendications 1 à 7, qui est une pâte dentifrice comprenant un abrasif calcique choisi parmi un sel de phosphate de calcium et un carbonate de calcium.

10. Composition selon la revendication 9, dans laquelle l'abrasif calcique comprend du pyrophosphate de calcium.

11. Composition selon l'une quelconque des revendications précédentes, comprenant en outre un agent antitartre tel que le pyrophosphate de tétrasodium.

12. Composition selon la revendication 11, comprenant les ingrédients suivants en poids :
a. Glycérine 0 à 7 %
b. Propylène glycol 50 à 65 %
c. Copolymère d'oxyde d'éthylène et d'oxyde de propylène, MW moy. > 1 kDa 5 à 15 %
d. Lauryl sulfate de sodium 1 à 3 %
e. Polyvinylpyrrolidone réticulée complexée avec 15 à 25 % d'H₂O₂5 à 12 %
f. Pyrophosphate de calcium 5 à 35 %
g. Pyrophosphate de tétrasodium 0,3 à 5 %

13. Composition selon l'une quelconque des revendications précédentes, comprenant 0,5 à 1 % en poids d'une source d'ions fluorure, par exemple du monofluorophosphate de sodium.

14. Composition selon l'une quelconque des revendications précédentes comprenant un aromatisant, un édulcorant, un antioxydant, un antimicrobien ou une combinaison de deux ou plus de ceux-ci.
